# EUROPÄISCHE PATENTSCHRIFT

(11) **EP 0 511 981 B1**
(45) Veröffentlichungstag und Bekanntmachung des Hinweises auf die Patenterteilung: **08.03.1995**
(21) Anmeldenummer: 91902086.7
(22) Anmeldetag: 15.01.1991
(51) Int. Cl.: C07C 69/24, C07C 67/303, C07C 53/126, C07C 51/36

(54) **GESÄTTIGTE, VERZWEIGTE FETTSÄUREN MIT 20 BIS 28 KOHLENSTOFFATOMEN BZW. ESTER DERSELBEN MIT C1-C36-ALKANOLEN, VERFAHREN ZU IHRER HERSTELLUNG UND IHRE VERWENDUNG**
SATURATED BRANCHED FATTY ACIDS WITH 20 TO 28 CARBON ATOMS OR ESTERS THEREOF WITH C1-C36 ALKANOLS, PROCESS FOR PRODUCING THEM AND THEIR USE
ACIDES GRAS SATURES, RAMIFIES COMPORTANT DE 20 A 28 ATOMES DE CARBONE OU LEURS ESTERS COMPORTANT DES ALCANOLES C1-C36, PROCEDE DE FABRICATION ET UTILISATION

(30) Priorität: 24.01.1990 DE 4002011
(43) Veröffentlichungstag der Anmeldung: 11.11.1992
(73) Patentinhaber: Henkel Kommanditgesellschaft auf Aktien, 40191 Düsseldorf (DE)
(72) Erfinder: LAUFENBERG, Alfred, D-4047 Dormagen 5 (DE); BEHR, Arno, D-4000 Düsseldorf 13 (DE)
(86) Internationale Anmeldenummer: EP9100053
(87) Internationale Veröffentlichungsnummer: WO9111426

(56) Entgegenhaltungen:
- EP-A- 0 010 807
- WO-A-84/00884
- FR-A- 2 202 727

## Beschreibung

Die Erfindung betrifft gesättigte, verzweigte Fettsäuren bzw. Ester derselben mit C₁-C₃₆-Alkanolen, erhältlich durch Hydrierung von olefinisch ungesättigten Addukten von Ethylen an mehrfach ungesättigte Fettsauren mit 18 bis 22 Kohlenstoffatomen bzw. an Ester derselben mit C₁-C₃₆-Alkanolen in molaren Verhältnissen von Ethylen zu Fettsäuren bzw. Fettsäureestern im Bereich von 1 : 1 bis 3 : 1.

### Stand der Technik

In der Alkylkette verzweigte Fettsäuren vom Typ der Guerbetsäuren, erhältlich durch Guerbetisierung der entsprechenden Fettalkohole und Oxidation der Guerbetalkohole zu den entsprechenden Säuren, sind technologisch interessante Zwischenprodukte, weil sie bzw. ihre Alkylester, im Vergleich zu den entsprechenden unverzweigten Isomeren, deutlich erniedrigte Stockpunkte aufweisen. Die Herstellung von Guerbetsäuren ist jedoch technologisch aufwendig und nur mit unbefriedigenden Ausbeuten durchführbar. Es hat daher nicht an Versuchen gefehlt, ausgehend von Fettsäuren bzw. Estern derselben entsprechende Fettsäurederivate herzustellen, die in der Alkylkette Verzweigungen aufweisen. Ein typisches Beispiel hierfür ist die schichtsilikatkatalysierte Fettsäuredimerisierung bei der jedoch auch erhebliche Mengen an trimeren Fettsäuren sowie an methylverzweigten Fettsäuren, sogenannten Isofettsäuren, gebildet werden.

Ein weiteres, wenn auch aufwendiges Verfahren liefert aus Konjuenfettsäuren in der trans,trans-Form mit aktivierten Dienophilen unter den Bedingungen einer Diels-Alder-Reaktion verzweigte Fettsäurederivate; so läßt sich z.B. auf diesem Wege aus Linolsäure und Acrylsäure eine verzweigte C₂₁-Dicarbonsäure herstellen, vgl. **US-B 3,734,859, US-B 3,753,968, DE-B 2 253 930**.

Des weiteren sind aus der **EP-A1 0 010 807** verzweigte Monocarbonsäuren bekannt, die durch Telomerisation von einem Mol Acetanhydrid und mindestens drei Mol eines Olefins mit mindestens 6 und bis zu 30 Kohlenstoffatomen in Gegenwart einer dreiwertigen Manganverbindung erhalten werden.

Weitere, verzweigte Fettsäurederivate sind durch thermische oder säurekatalysierte Addition von aktivierten Enophilen an ungesättigte Fettsäurederivate erhalten worden. So läßt sich z.B. Maleinsäureanhydrid unter Säurekatalyse mit bis zu 70 % Ausbeute an Ölsäure addieren, vgl. **Fat. Sci. Technol., 1, 1 (1988)**. Bei den vorstehend genannten Reaktionsprodukten erweist sich die Anwesenheit von mehr als einer Carboxylgruppe jedoch häufig als störend.

Schließlich hat man auch versucht, gesättigte Kohlenwasserstoffe durch thermisch initiierte radikalische Addition von gesättigten Kohlenwasserstoffen an Fettsäuren anzulagern; die Addition von Cyclohexan an Ölsäuremethylester führt bei 340°C und 200 bar mit 70%-iger Selektivität, jedoch mit einer Ausbeute von lediglich 2,8 %, zu alkylverzweigten Fettsäuren, **vgl. J.O. Metzger, et al., Fat. Sci. Technol. 1 (1989), 18**.

### Beschreibung der Erfindung

Gegenstand der Erfindung sind gesättigte verzweigte Fettsäuren bzw. Ester derselben mit C₁-C₃₆-Alkanolen, die man dadurch erhält, daß man
a) ungesättigte Fettsäuren mit 18 bis 22 Kohlenstoffatomen und mehr als einer olefinischen Doppelbindung bzw. Ester derselben mit C₁-C₃₆-Alkanolen bei erhöhter Temperatur und erhöhtem Druck in Gegenwart von Verbindungen von Übergangsmetallen aus der von Ru, Rh, Pd, Ir und Pt gebildeten Gruppe mit Ethylen umsetzt, wobei das molare Verhältnis von Ethylen zu Fettsäure bzw. Fettsäureester 1 : 1 bis 3 : 1 beträgt, und
b) die resultierenden olefinisch ungesättigten Addukte bei einer Temperatur im Bereich von 70 bis 120°C und einem Wasserstoffdruck im Bereich von 10 bis 30 bar in Gegenwart von Hydrierkatalysatoren hydriert.

Die gesättigten, verzweigten Fettsäuren bzw. Ester derselben können auf einfache Weise und mit guten Ausbeuten hergestellt werden. Die erfindungsgemäß bereitgestellten Verbindungen sind neue Produkte, die sich z.B. von in der Natur vorkommenden ethylverzweigten Fettsäuren mit insgesamt 12 bis 18 Kohlenstoffatomen, beschrieben in **A. Smith et al, Biomed. Mass Spectrom., 6 (8), 347-349**, bereits durch ihre Kettenlänge unterscheiden.

### Ausgangsstoffe

Die gesättigten, verzweigten Fettsäuren der Erfindung bzw. Ester derselben können durch Hydrierung von olefinisch ungesättigten Addukten von Ethylen an mehrfach ungesättigte Fettsäuren mit 18 bis 22 Kohlenstoffatomen bzw. an Ester derselben mit C₁-C₃₆-Alkanolen in molaren Verhältnissen von Ethylen zu Fettsäuren bzw. Fettsäureestern im Bereich von 1:1 bis 3:1 erhalten werden.

Diese Ethylenaddukte sind Gegenstand der zeitgleichen Patentanmeldung **DE-A1-40 02 012** der Anmelderin, entsprechend **EP-A-0511 986**, auf die hier ausdrücklich Bezug genommen und deren wesentlicher Inhalt im folgenden zusammengefaßt ist :
Als Ausgangsprodukte zur Herstellung der olefinisch ungesättigten Addukte der genannten Patentanmeldung eignen sich ungesättigte Fettsäuren mit 18 bis 22 Kohlenstoffatomen und mehr als einer olefinischen Doppelbindung wie Linolsäure, isomerisierte Linolsäure mit konjuierten Doppelbindungen (sogenannte C18:2-Konjuenfettsäure), Linolensäure, Arachidonsäure, Docosadiensäure, Docosahexaensäure und Eicosapentaensäure, die in Form ihrer technischen Gemische mit anderen Fettsäuren aus nachwachsenden natürlichen Rohstoffen, z.B. aus Sonnenblumenöl, Tallöl oder Fischöl, erhalten werden können. Diese mehrfach ungesättigten Fettsäuren werden, wie in der Fettchemie üblich, im allgemeinen nicht in Form ihrer reinen Verbindungen, sondern in Form ihrer technischen Gemische zur Herstellung der Addukte eingesetzt. Die vorgenannten Fettsäuren werden bevorzugt nicht nur als solche, sondern auch in Form ihrer Ester mit C₁-C₃₆-Alkanolen, insbesondere mit C₁-C₄-Alkanolen, eingesetzt. Typische Beispiele für derartige Alkanole zur Bildung von Estern mit den vorgenannten Fettsäuren sind Methanol, Ethanol, Propanol, Butanol, Pentanol, Hexanol, Octanol, Decanol, Dodecanol, Tetradecanol, Hexadecanol, Octadecanol sowie höhere Fettalkohole bzw. Fettalkoholderivate mit bis zu 36 Kohlenstoffatomen, z.B. C₃₆-Guerbetalkohole.

### Katalysatoren

Die vorgenannten mehrfach ungesättigten Fettsäuren bzw. Fettsäureester lagern gemäß der genannten Patentanmeldung bei erhöhter Temperatur und erhöhtem Druck in Gegenwart von Verbindungen von Übergangsmetallen aus der von Ru, Rh, Pd, Ir und Pt gebildeten Gruppe Ethylen an.

Typische Beispiele für dabei einsetzbare Katalysatoren sind die folgenden:
RhCl₃.3H₂O
RhBr₃.3H₂O
[(C₂H₄)₂RhCl]₂
Rh(NO₃)₃.2H₂O
Rh(OOCCH₃)₂.2H₂O
Rh(acetylacetonat)₃
RhF₃.6H₂O
RhJ₃
Rh(CN)₃.3H₂O
Rh₂(SO₄)₃
Rh₂(CO₃)₃
[(1.5-Cyclooctadien)RhCl]₂
[(C₂H₄)₂Rh(acetylacetonat)]
[(1.3-Butadien)RhCl]₂
Cyclopentadienyl-Olefin-Komplexe wie [(n-C₅H₅)Rh(C₂H₄)₂].

Sofern die einsetzbaren Katalysatoren in wasserfreier Form vorliegen, kann es zweckmäßig sein, dem Reaktionsgemisch eine geringe Menge von Wasser zuzusetzen.

Die im Rahmen der genannten Patentanmeldung einsetzbaren Katalysatoren sind als solche für die Anlagerung von Ethylen an Alkadiene bekannt, vgl. **US-B 3,636,122; M. Bochmann et al., Journal of Molecular Catalysis, 22 (1984) 363-365; G. Wilkinson (Ed.), Comprehensive Organometallic Chemistry, S. 414-429, Pergamon Press (1982); A.C.L. Su, Advances in Organometallic Chemistry, Vol. 17, S. 271-283**; wobei diese Veröffentlichungen sich jedoch nicht mit der Anlagerung von Alkenen an Fettsäuren bzw. Fettsäurederivate oder andere Fettstoffe befassen.

Weitere, im Verfahren der genannten Patentanmeldung einsetzbare Katalysatoren sind z.B.
PdCl₂
PtCl₂
IrCl₃
Ru(acetylacetonat)₃.

### Liganden

Mit den vorgenannten Katalysatoren entstehen im allgemeinen Gemische von 1:1-, 2:1- und 3:1-Addukten von Ethylen an die Fettsäuren bzw. Fettsäureester. Durch Veränderung der Reaktionsbedingungen wie Druck, Temperatur und Reaktionszeit lassen sich jedoch die Anteile an den verschiedenen Addukten variieren. Setzt man dem Reaktionsgemisch jedoch neben den Katalysatoren geeignete Phosphin- oder Phosphitliganden, z.B.
P(C₄H₉)₃
P(OC₄H₉)₃
P(C₆H₅)₃
P(OC₆H₅)₃
oder andere, aus dem zuletzt diskutierten Stand der Technik sowie der **DE-B 20 16 133** bekannte Liganden zu, kann man unter Umständen gezielt die Zusammensetzung der Adduktgemische beeinflussen; so werden bei Reaktionssystemen, die ohne derartige Liganden überwiegend 2:1-Addukte liefern, beim Einsatz großer Liganden überwiegend 3:1-Addukte und beim Einsatz kleiner Liganden überwiegend 1:1-Addukte gebildet. Ähnliche Effekte lassen sich zum Teil dadurch erreichen, daß man dem Reaktionssystem Promotoren wie LiCl, FeCl₃ oder AgBF₄ zusetzt, die als solche ebenfalls aus dem zuletzt genannten Stand der Technik bekannt sind.

### Herstellung und Struktur der Ausgangsstoffe

Die Struktur der in der genannten Patentanmeldung beschriebenen olefinisch ungesättigten Addukte ist nicht einheitlich. Im Falle der Linolsäure (bzw. der von dieser abgeleiteten C₁₈-Konjuenfettsäure) konnte gezeigt werden, daß die Anlagerung des ersten Ethylenmoleküls zwischen den Positionen 9 und 12 der Kohlenstoffkette der Linolsäure erfolgt, wobei das 1:1-Addukt die gleiche Anzahl von Doppelbindungen wie die als Ausgangsmaterial eingesetzte Fettsäure aufweist. Die Stellung der Doppelbindungen ist jedoch unsicher. Die Doppelbindungen sind in keinem Fall mehr als 4 Kohlenstoffatome von der Verzweigung entfernt und stehen grundsätzlich in alpha,delta- oder in alpha,gamma-Stellung zueinander. Das zweite und gegebenenfalls das dritte Ethylenmolekül wird dann an eine in der Verzweigung befindliche Doppelbindung angelagert. Es ist zu vermuten, daß die erhaltenen Addukte mindestens teilweise eine der nachfolgend wiedergegebenen Strukturen aufweisen; bei den gemäß der vorliegenden Erfindung erhaltenen gesättigten Verbindungen liegen analoge Kohlenstoffgerüste vor.

Gemäß einer Ausführungsform der genannten Patentanmeldung weisen die gegebenenfalls in Form ihrer Ester eingesetzten mehrfach ungesättigten Fettsäuren 2 bis 5, insbesondere 2 bis 3 olefinische Doppelbindungen auf. 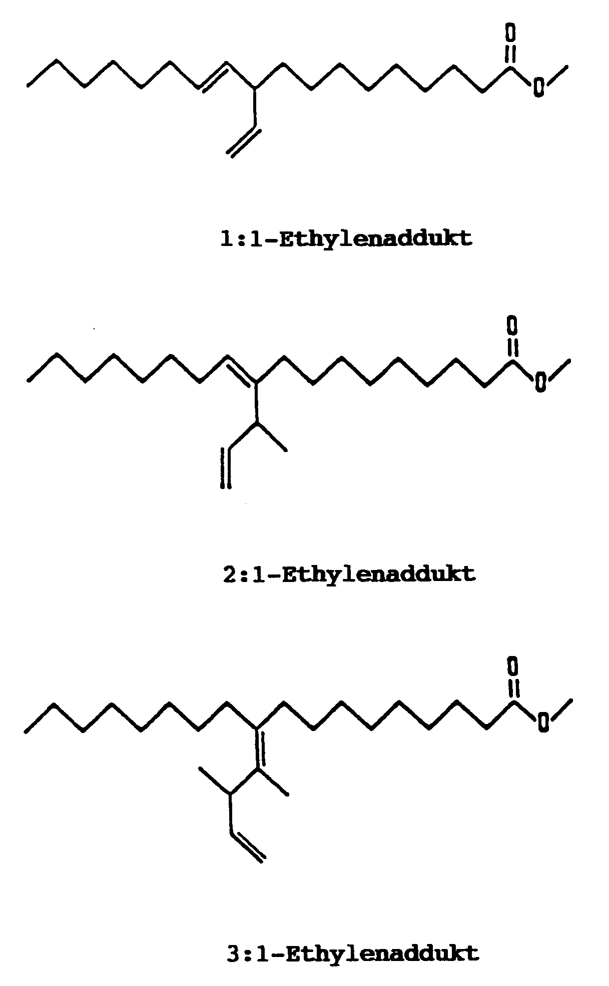
Gemäß einer weiteren Ausführungsform der genannten Patentanmeldung erhält man die Addukte bei einem Ethylendruck im Bereich von 5 bis 60 bar und einer Temperatur im Bereich von 50 bis 140°C, wobei man gegebenenfalls in Anwesenheit inerter organischer Lösemittel wie Hexan, Chloroform oder dergleichen, arbeitet.

Gemäß einer weiteren Ausführungsform der genannten Patentanmeldung verwendet man die Katalysatoren in einer Menge im Bereich von 0,02 bis 2 Mol-%, bezogen auf Fettsäuren bzw. Fettsäureester.

Vorteilhafterweise verwendet man gemäß der genannten Patentanmeldung als Katalysatoren Rhodiumverbindungen, wobei wiederum Rhodiumverbindungen aus der von RhCl₃ und RhBr₃ (einschließlich Hydraten derselben) sowie [(C₂H₄)₂RhCl]₂ gebildeten Gruppe als Katalysatoren bevorzugt sind.

### Erfindungsgemäßes Verfahren

Ein weiterer Gegenstand der Erfindung betrifft ein Verfahren zur Herstellung gesättigter verzweigter Fettsäuren bzw. Ester derselben mit C₁-C₃₆-Alkanolen, das sich dadurch auszeichnet, daß man
a) ungesättigte Fettsäuren mit 18 bis 22 Kohlenstoffatomen und mehr als einer olefinischen Doppelbindung bzw. Ester derselben mit C₁-C₃₆-Alkanolen bei erhöhter Temperatur und erhöhtem Druck in Gegenwart von Verbindungen von Übergangsmetallen aus der von Ru, Rh, Pd, Ir und Pt gebildeten Gruppe mit Ethylen umsetzt, wobei das molare Verhältnis von Ethylen zu Fettsäure bzw. Fettsäureester 1 : 1 bis 3 : 1 beträgt, und
b) die resultierenden olefinisch ungesättigten Addukte bei einer Temperatur im Bereich von 70 bis 120°C und einem Wasserstoffdruck im Bereich von 10 bis 30 bar in Gegenwart von Hydrierkatalysatoren hydriert.

### Gewerbliche Anwendbarkeit

Schließlich betrifft die Erfindung die Verwendung der gesättigten, verzweigten Fettsäuren bzw. Ester derselben als Stockpunktserniedriger in Schmiermitteln.

Die Erfindung wird im folgenden anhand bevorzugter Ausführungsbeispiele näher erläutert.

### Beispiele

### I. Herstellung der Ausgangsstoffe

Zunächst wird in den nichterfindungsgemäßen Herstellbeispielen H1 bis H13 die Herstellung der Ausgangsverbindungen für die Verbindungen der Erfindung gemäß **DE-A1-40 02 012** beschrieben.
H1) 8,2 g eines technischen Fettsäuregemisches mit einem Anteil von 56 Gew.-% konjugierter C18:2-Fettsäure wurden mit 100 mg RhCl₃.3H₂O in 10 ml Hexan 20 h bei 100°C und 30 bar Ethylenkaltdruck in einem 75 ml-Stahlautoklaven umgesetzt. Man erhielt (gaschromatografisch ermittelt) 63,1 % Ethylenaddukte, bezogen auf Konjuenfettsäure, davon 34,9 % 1:1-, 44,2 % 2:1- und 20,9 % 3:1-Addukte.
H2) 2,5 kg einer technischen Konjuenfettsäure gemäß H1), 0,8 g RhCl₃.3H₂O und 1 l Hexan wurden 20 h bei 100°C und 20 bar konstantem Ethylendruck in einem 5 l-Rührautoklaven mit Turbinenrührer umgesetzt. Die Ausbeute an Ethylenaddukten betrug 89,4 %, davon 23,7 % 1:1-, 41,7 % 2:1- und 34,6 % 3:1-Addukte.
H3) 8,2 g eines technischen Linolsäuremethylesters (67,8 % Methyllinolat, 22,4 % Methyloleat) und 100 mg RhCl₃.3H₂O wurden mit 10 ml Chloroform bei 100°C und einem Ethylenkaltdruck von 20 bar umgesetzt. Die Adduktausbeute, bezogen auf Methyllinolat, betrug 57,8 %, davon 30,8 % 1:1-, 42,0 % 2:1- und 27,2 % 3:1-Addukte.
H4) Die Wiederholung von H3) bei einem Ethylenkaltdruck von 30 bar ergab eine Adduktausbeute von 57,6 % bei Anteilen von 31,5 % 1:1-, 52,2 % 2:1- und 16,3 % 3:1-Addukten.
H5) 8,2 g eines technischen C18:2-Konjuenmethylesters (enthaltend 60,3 % Konjuenester, 6,2 % Linolsäuremethylester und 24,4 % Ölsäuremethylester) in 10 ml Chloroform wurden bei 100°C und 20 bar Ethylenkaltdruck umgesetzt. Man erhielt 93,5 % Ethylenaddukte, davon 27,5 % 1:1-, 51,1 % 2:1- und 21,4 % 3:1-Addukte.
H6) Die Wiederholung von H5) bei einem Ethylenkaltdruck von 30 bar ergab eine Gesamtausbeute an Ethylenaddukten von 88,5 %, davon 36,5 % 1:1-, 51,0 % 2:1- und 12,1 % 3:1-Addukte.
H7) Die Wiederholung von H6) unter Zusatz von 185,1 mg FeCl₃ als Promotor ergab eines Gesamtadduktausbeute von 81,8 %, davon 22,8 % 1:1-, 46,9 % 2:1- und 30,3 % 3:1-Addukte.
H8) Die Wiederholung von H6) mit Hexan anstelle von Chloroform ergab eine Gesamtadduktausbeute von 87,4 %, davon 24,8 % 1:1-, 59,7 % 2:1- und 15,5 % 3:1-Addukte.
H9) Die Wiederholung von H6) mit 74 mg [(C₂H₄)₂RhCl]₂ anstelle von RhCl₃.3H₂O ergab eine Gesamtadduktausbeute von 78,0 %, davon 24,2 % 1:1-, 61,8 % 2:1- und 13,0 % 3:1-Addukte. Die Gesamtausbeute stieg auf 86,5 %, wenn man dem Reaktionsgemisch 86,4 mg P(C₄H₉)₃ zusetzte.
H10) Die Wiederholung von H6) unter Verwendung von 13 mg RhBr₃.3H₂O anstelle von RhCl₃.3H₂O sowie in Abwesenheit eines Lösemittels ergab eine Gesamtausbeute an Ethylenaddukten, bezogen auf Konjuenestergehalt, von 94,6 %, davon 17,7 % 1:1-, 62,8 % 2:1- und 16,5 % 3:1-Addukte.
H11) Einfluß von Phosphin- und Phosphitliganden. H3) wurde unter Zusatz von 76,76 (0,380 mmol) P(C₄H₉)₃ wiederholt. Die Gesamtausbeute an Addukt betrug 54,5 %, bezogen auf Methyllinolat, davon 32,4 % 1:1-, 16,2 % 2:1- und 6,0 % 3:1-Addukt.
Die Wiederholung mit 9,50 mg (0,038 mmol) P(OC₄H₉)₃ ergab eine Gesamtadduktausbeute von 57,8 %, davon 34,9 % 1:1-, 16,4 % 2:1- und 6,5 % 3:1-Addukt.
Die Wiederholung mit 9,96 mg (0,038 mmol) P(C₆H₅)₃ ergab eine Gesamtadduktausbeute von 64,2 %, davon 8,7 % 1:1-, 17,9 % 2:1- und 37,5 % 3:1-Addukt.
Die Wiederholung mit 117,8 mg (0,38 mmol) P(OC₆H₅)₃ ergab eine Gesamtadduktausbeute von 59,2 % bei ähnlicher Adduktverteilung wie bei P(C₆H₅)₃.
H12) H8) wurde mit verschiedenen Katalysatoren anstelle des dort beschriebenen Katalysators wiederholt. In der folgenden Tab.1 sind die eingesetzten Katalysatormengen (jeweils 0,38 mmol), die Art des eingesetzten Katalysators und die Adduktausbeuten (jeweils bezogen auf Konjuenester) angegeben:

**Tab.1**

| Katalysator und Adduktausbeute | | |
|---|---|---|
| Katalysator | Menge mg | Adduktausbeute %-rel |
| Rh(NO₃)₃ * 2H₂O | 12,3 | 17,6 |
| [Rh(OAc)₂] * 2H₂O | 490,8 | 6,6 |
| Rh(acac)₃# | 161,1 | 44,1 |
| Ru(acac)₃# | 51,4 | 17,3 |
| PdCl₂ | 67,4 | 14,8 |
| PtCl₂ | 101,1 | 32,3 |
| IrCl₃# | 113,5 | 13,6 |

| | | |
|---|---|---|
| #) + 2 »l H₂O | | |

Es entstanden ausschließlich 1:1-Addukte.
H13) Ein C₃₆-Guerbetester einer technischen C18:2-Konjuenfettsäure (8,2 g) der in H1) angegebenen Zusammensetzung wurde unter den dort angegebenen Reaktionsbedingungen mit Ethylen in Gegenwart von 100 mg RhCl₃*3H₂O und 10 ml Hexan umgesetzt. Die Gesamtausbeute an Addukten betrug 52,4 %, bezogen auf Konjuenester, davon 16,4 % 1:1-, 27,0 % 2:1- und 9,2 % 3:1-Addukte.

### II. Erfindungsgemäße Beispiele

Die vorstehend erläuterten Ausgangsmaterialien lassen sich erfindungsgemäß zu gesättigten, verzweigten Fettsäuren mit 20 bis 28 Kohlenstoffatomen bzw. Estern derselben mit C₁-C₃₆-Alkanolen hydrieren. Hierfür werden im folgenden bevorzugte Ausführungsbeispiele angegeben.

### Beispiel 1:

Ein Ausgangsmaterial gemäß H1) wurde unter Zugabe von 1 mol-%, bezogen auf Palladium, eines 5 Gew.-% Palladium auf Aktivkohle enthaltenden Katalysators bei 70°C und 10 bar Wasserstoffdruck vollständig gehärtet. Die gehärtete Reaktionsmischung enthielt neben den hydrierten Addukten und den schon im Ausgangsmaterial enthaltenen Verunreinigungen ausschließlich Stearinsäure. Die gehärteten Additionsprodukte konnten durch Destillation in hoher Reinheit erhalten werden.

### Beispiel 2:

Ein Ausgangsmaterial gemäß H2) wurde in der in Beispiel 1 beschriebenen Weise gehärtet. Man erhielt das gewünschte Gemisch von hydrierten Addukten der analogen Zusammensetzung.

### Beispiel 3:

Ein überwiegend 1:1-Addukte von Ethylen an C18:2-Konjuenmethylester enthaltendes Produkt wurde in der in Beispiel 1 beschriebenen Weise gehärtet. Aus dem Reaktionsgemisch wurde durch Molekulardestillation das gehärtete 1:1-Addukt in hoher Reinheit (ca. 98 %, gaschromatografisch ermittelt) erhalten. Das wasserklare, farblose Destillat hatte einen besonders niedrigen Stockpunkt von -58°C. Dieser Stockpunkt lag wesentlich niedriger als z.B. derjenige des Isostearinsäuremethylesters (ca. -15°C).

## Patentansprüche

1. Gesättigte verzweigte Fettsäuren bzw. Ester derselben mit C₁-C₃₆-Alkanolen, dadurch erhältlich, daß man
a) ungesättigte Fettsäuren mit 18 bis 22 Kohlenstoffatomen und mehr als einer olefinischen Doppelbindung bzw. Ester derselben mit C₁-C₃₆-Alkanolen bei erhöhter Temperatur und erhöhtem Druck in Gegenwart von Verbindungen von Übergangsmetallen aus der von Ru, Rh, Pd, Ir und Pt gebildeten Gruppe mit Ethylen umsetzt, wobei das molare Verhältnis von Ethylen zu Fettsäure bzw. Fettsäureester 1 : 1 bis 3 : 1 beträgt, und
b) die resultierenden olefinisch ungesättigten Addukte bei einer Temperatur im Bereich von 70 bis 120°C und einem Wasserstoffdruck im Bereich von 10 bis 30 bar in Gegenwart von Hydrierkatalysatoren hydriert.

2. Verfahren zur Herstellung gesättigter verzweigter Fettsäuren bzw. Ester derselben mit C₁-C₃₆-Alkanolen, **dadurch gekennzeichnet**, daß man
a) ungesättigte Fettsäuren mit 18 bis 22 Kohlenstoffatomen und mehr als einer olefinischen Doppelbindung bzw. Ester derselben mit C₁-C₃₆-Alkanolen bei erhöhter Temperatur und erhöhtem Druck in Gegenwart von Verbindungen von Übergangsmetallen aus der von Ru, Rh, Pd, Ir und Pt gebildeten Gruppe mit Ethylen umsetzt, wobei das molare Verhältnis von Ethylen zu Fettsäure bzw. Fettsäureester 1 : 1 bis 3 : 1 beträgt, und
b) die resultierenden olefinisch ungesättigten Addukte bei einer Temperatur im Bereich von 70 bis 120°C und einem Wasserstoffdruck im Bereich von 10 bis 30 bar in Gegenwart von Hydrierkatalysatoren hydriert.

3. Verwendung der gesättigten, verzweigten Fettsäuren bzw. der Ester gemäß Anspruch 1 als Stockpunktserniedriger in Schmiermitteln.

## Claims

1. Saturated branched fatty acids or esters thereof with C₁₋₃₆ alkanols obtainable by
a) reaction of unsaturated fatty acids containing 18 to 22 carbon atoms and more than one olefinic double bond or esters thereof with C₁₋₃₆ alkanols with ethylene at elevated temperature and pressure in the presence of compounds of transition metals from the group consisting of Ru, Rh, Pd, Ir and Pt, the molar ratio of ethylene to fatty acid or fatty acid ester being from 1:1 to 3:1, and
b) hydrogenation of the resulting olefinically unsaturated adducts in the presence of hydrogenation catalysts at a temperature of 70 to 120°C and under a hydrogen pressure of 10 to 30 bar.

2. A process for the production of saturated branched fatty acids or esters thereof with C₁₋₃₆ alkanols, characterized in that
a) unsaturated fatty acids containing 18 to 22 carbon atoms and more than one olefinic double bond or esters thereof with C₁₋₃₆ alkanols are reacted with ethylene at elevated temperature and pressure in the presence of compounds of transition metals from the group consisting of Ru, Rh, Pd, Ir and Pt, the molar ratio of ethylene to fatty acid or fatty acid ester being from 1:1 to 3:1, and
b) the resulting olefinically unsaturated adducts are hydrogenated in the presence of hydrogenation catalysts at a temperature of 70 to 120°C and under a hydrogen pressure of 10 to 30 bar.

3. The use of the saturated branched fatty acids or esters claimed in claim 1 as pour point depressants in lubricants.

## Revendications

1. Acides gras saturés, ramifiés ou les esters de ceux-ci avec des alcanols en C₁ à C₃₆ qui sont accessibles en ce que :
a) l'on fait réagir des acides gras non saturés ayant de 18 à 22 atomes de carbone et plus d'une double liaison oléfinique, ou des esters de ceux-ci avec des alcanols en C₁ à C₃₆, à une température élevée et à une pression poussée, avec de l'éthylène en présence de dérivés des métaux de transition choisis dans le groupe formé de Ru, Rh, Pd, Ir et Pt, réaction dans laquelle le rapport molaire de l'éthylène aux acides gras ou aux esters d'acide gras s'élève de 1 : 1 à 3 : 1,
et b) on hydrogène les produits d'addition non saturés oléfiniquement résultants, à une température dans la plage de 70 à 120°C et à une pression d'hydrogène dans la zone de 10 à 30 bar en présence de catalyseurs d'hydrogénation.

2. Procédé d'obtention d'acides gras saturés, ramifiés, ou d'esters de ceux-ci avec des alcanols en C₁ à C₃₆, caractérisé en ce que :
a) on fait réagir des acides gras non saturés ayant de 18 à 22 atomes de carbone et plus d'une double liaison ou bien des esters de ceux-ci avec des alcanols en C₁ à C₃₆, à température élevée et à pression accrue, avec de l'éthylène en présence de dérivés de métaux de transition choisis dans le groupe formé de Ru, Rh, Pd, Ir, et Pt, réaction dans laquelle le rapport molaire de l'éthylène aux acides gras ou aux esters d'acide gras s'élève de 1 : 1 à 3 : 1, et
b) on hydrogène les produits d'addition non saturés oléfiOniquement résultants à une température dans la plage allant de 70 à 120°C et à une pression d'hydrogène dans la zone de 10 à 30 bar en présence de catalyseurs d'hydrogénation.

3. Utilisation d'acides gras saturés, ramifiés ou d'esters selon la revendication 1, comme agents d'abaissement du point de solidification dans des agents lubrifiants.
